# EUROPEAN PATENT APPLICATION

(11) **EP 3 348 306 A1**
(43) Date of publication of application: **18.07.2018**
(21) Application number: 17156692.0
(22) Date of filing: 17.02.2017
(51) Int. Cl.: A61N 1/375, A61N 1/372, A61N 1/39

(54) **CARDIAC PACING SYSTEM COMPRISING AN IMPLANTABLE LEADLESS PACEMAKER AND AN IMPLANTABLE CARDIOVERTER-DEFIBRILLATOR**

(30) Priority: 17.01.2017 US 201762446846 P
(71) Applicant: BIOTRONIK SE & Co. KG, 12359 Berlin (DE)
(72) Inventor: Müssig, Dirk, West Linn, OR Oregon 97068 (US); Stotts, Larry, Tigard, OR Oregon 97224 (US)
(74) Representative: Keck, Hans-Georg

(57) **Abstract**

The present invention relates to a cardiac pacemaking system (1), comprising: an implantable leadless pacemaker (10) that is configured to be implanted into a chamber of a patient's heart (2), and an implantable subcutaneous cardioverter-defibrillator (20), wherein the leadless pacemaker (10) is configured to apply electrical pacing pulses to the heart (2), particularly in order to provide antibradycardia pacing, and wherein the subcutaneous cardioverter-defibrillator (20) is configured to apply electrical stimulation to the heart (2) for providing defibrillation of the heart (2), and wherein the leadless pacemaker (10) is configured to sense the patient's heart rhythm, and wherein the cardiac pacemaking system (1) comprises a communication link (101) between the leadless pacemaker (10) and the subcutaneous cardioverter-defibrillator (20), wherein the leadless pacemaker (10) is configured to send an output signal to the subcutaneous cardioverter-defibrillator (20) via said communication link (101) in an unidirectional fashion when the leadless pacemaker (10) detects a heart rhythm that requires said electrical stimulation of the subcutaneous cardioverter-defibrillator (20), and wherein the subcutaneous cardioverter-defibrillator (20) is configured to apply said electrical stimulation to the heart (2) when the subcutaneous cardioverter-defibrillator (20) receives said output signal.

## Description

The invention relates to a cardiac pacemaker system comprising an implantable leadless pacemaker as well as a subcutaneous implantable cardioverter-defibrillator (ICD).

Such a pacemaker system provides the functionality of cardiac pacemaking via the leadless pacemaker when the heart's natural pacemaker or conduction system cannot e.g. provide synchronized atrial and ventricular contractions at appropriate intervals (e.g. due to bradycardia).

On the other hand, the subcutaneous implantable cardioverter-defibrillator may be configured to reset the activity of the heart of the patient by providing a high voltage shock to the heart, e.g. in case of a cardiac arrest.

US 2014/0309706 A1 discloses a cardiac pacing system comprising one or more leadless cardiac pacemakers configured for implantation in electrical contact with a cardiac chamber and configured to perform cardiac pacing functions in combination with a co-implanted implantable cardioverter-defibrillator (ICD). The leadless cardiac pacemaker comprises at least two leadless electrodes configured for delivering cardiac pacing pulses, sensing evoked and/or natural cardiac electrical signals, and bidirectionally communicating with the co-implanted ICD.

The development of leadless pacemakers requires making the devices as small as possible to be able to fit into the apex of the right ventricle of the heart. To achieve this goal such a device is using battery sizes being significantly smaller than existing conventional pacemakers. Therefore, leadless pacemakers are limited in functionality like the capability to transmit clinical information over a wide range (Home Monitoring), or to provide high voltage therapy like ICDs. On the other hand, subcutaneous ICD do not comprise any leads going directly to the myocardium. Therefore, such devices are limited in the delivery of low voltage therapy (e.g. pacing, ATP, post shock pacing). Also since there are no leads going to the heart, S-ICDs are also limited in their sensing capabilities and are susceptible to false sensing due to myopotentials.

Thus, the problem underlying the present invention is to provide a cardiac pacemaking system that reduces the above-stated difficulties.

This objective is solved by means of a cardiac pacemaking system having the features of claim 1. Embodiments of this aspect of the present invention are stated in the sub claims or are described below.

According to claim 1 a cardiac pacemaking system is disclosed, comprising: an implantable leadless pacemaker that is configured to be implanted into a chamber of a patient's heart, and an implantable subcutaneous cardioverter-defibrillator, wherein the leadless pacemaker is configured to apply electrical (e.g. low voltage) pacing pulses to the heart (e.g. to said chamber), particularly in order to provide antibradycardia pacing, and wherein the subcutaneous cardioverter-defibrillator is configured to apply (e.g. high voltage) electrical stimulation to the heart for providing defibrillation of the heart (i.e. shock therapy), and wherein the leadless pacemaker is configured to sense the patient's heart rhythm, and wherein the cardiac pacemaking system is configured to establish an unidirectional communication link between the leadless pacemaker and the subcutaneous cardioverter-defibrillator, wherein the leadless pacemaker is configured to send an output signal to the subcutaneous cardioverter-defibrillator via said communication link in an unidirectional fashion when the leadless pacemaker detects a heart rhythm that requires said electrical stimulation of the subcutaneous cardioverter-defibrillator, and wherein the subcutaneous cardioverter-defibrillator is configured to apply said electrical stimulation (e.g. shock therapy) when the subcutaneous cardioverter-defibrillator receives said output signal.

Particularly, in an embodiment, in case the leadless pacemaker detects a heart rhythm that cannot be treated via pacing pulses provided by the leadless pacemaker (e.g. low voltage therapy) and further determines that the detected heart rhythm cannot provide cardiac output required to sufficiently perfuse organs (e.g. the heart or brain of the patient) it communicates said output signal to said subcutaneous ICD to perform said electrical stimulation (e.g. high voltage therapy/delivery of shock).

Herein, low voltage particularly means that the electrical pacing pulses comprise an amplitude in the range from 0.2 Volt to 7.5 Volt. Further, herein, high voltage electrical stimulation means that the applied electrical stimulation comprises an amplitude in the range from 6 Joule to 80 Joule.

Particularly, in an embodiment, the leadless pacemaker is configured to be implanted into a ventricle of the patient's heart, particularly into the right or left ventricle, particularly via a catheter. The leadless pacemaker may comprise a hermetically sealed housing enclosing a pulse generator for generating said pacing pulses and a battery for supplying energy to the pulse generator. The leadless pacemaker may further comprise fastening means provided on the housing for fastening the leadless pacemaker to the chamber/ventricle, and pacing electrodes (e.g. a cathode and an anode) provided e.g. on opposite sides of the housing of the leadless pacemaker for applying the electrical stimulation to the heart. Regarding the pacemaker the notion leadless means that the electrodes of the leadless pacemaker are not connected to a subcutaneous housing via a transvenous lead but are provided on or directly coupled to the housing. Such a leadless pacemaker is for instance disclosed in US 2014/0303704 A1 which is incorporated herein its entirety by reference.

The proposed system containing a leadless pacemaker device that is capable to sense, classify and provide low voltage pacing treatment and said subcutaneous ICD providing high voltage treatment (and particularly providing a RF link to a remote server, see below) is advantageous since the individual components provide a more targeted diagnosis and therapy. Also such a system is advantageous since the therapy options are "upgradable", e.g. a patient requiring bradycardia therapy can be implanted with the leadless pacemaker initially. As the heart disease might worsen, requiring the addition of a high voltage therapy system, it is not required to explant the low voltage system, but the e.g. subcutaneous ICD can be implanted in a second procedure.

Further, the specific communication configuration (unidirectional output signals from the leadless pacemaker to the subcutaneous ICD) further completely eliminates a requirement for individual heart rate sensing and discrimination by the subcutaneous ICD. This is especially beneficial for the subcutaneous ICD since (due to the sensing lead of an ICD being normally implanted in a subcutaneous location) the sensing accuracy is significantly more challenging due to local motion artifacts and muscle noise. Particularly, in contrast, in the framework of the present invention, the subcutaneous ICD ideally doesn't need any heart beat sensing capabilities but is merely listening to commands from the implantable leadless pacemaker.

Combining the two different therapy systems as proposed by the present invention allows providing full therapy while the drawbacks of each individual system are compensated by the respective other system. Also, the combination of both systems allows upgrading a low voltage therapy system (e.g. leadless pacemaker) without requiring explant.

Further, according to an embodiment of the present invention, the subcutaneous cardioverter-defibrillator is configured to merely receive information about the patient's heart via said unidirectional communication link. Particularly, the subcutaneous cardioverter-defibrillator does not comprise sensing means on its own for sensing heart parameters such as heart rhythm etc., but exclusively relies on data provided by the leadless pacemaker (i.e. said unidirectional communication from the leadless pacemaker). Particularly said output signal, is the only information available to the subcutaneous cardioverter-defibrillator from the patient's heart.

Further, according to an embodiment of the present invention, the leadless pacemaker is further configured to provide heart rhythm classification of a sensed heart rhythm into different classes, wherein a class may represent one of: a heart rate that is too low, requiring electrical pacing pulses from the leadless pacemaker, a heart rate that is normal, no therapy required, and a heart rate that is too high, requiring electrical therapy by the subcutaneous cardioverter-defibrillator.

Furthermore, according to an embodiment of the present invention, the leadless pacemaker is further configured to identify a source of a heart rhythm disturbance, particularly whether said heart rhythm disturbance originates from atrial or ventricular arrhythmia.

Furthermore, according to an embodiment of the present invention, the leadless pacemaker is configured to apply (e.g. low voltage) electrical pacing pulses to the heart for providing at least one of
- antibradycardia pacing,
- anti-tachycardia pacing (ATP)
- post shock pacing.

Furthermore, according to an embodiment of the present invention, the leadless pacemaker is configured to measure cardiac output (e.g. by using an accelerometer to identify ventricular motion)

Further, according to an embodiment of the present invention, the subcutaneous cardioverter-defibrillator is configured to transmit data to a remote server via a further communication link, particularly via a radio frequency (RF) link (e.g. to provide remote monitoring functionalities).

Further, according to an embodiment of the present invention, the subcutaneous cardioverter-defibrillator is configured to transmit said data to said remote server upon request of the leadless pacemaker, which is configured to transmit said request to the subcutaneous cardioverter-defibrillator via said communication link.

Further, according to an embodiment of the present invention, said data is at least one of: data about electrical stimulation applied to the heart by the leadless pacemaker and/or the subcutaneous cardioverter-defibrillator, status information on the leadless pacemaker and/or on the subcutaneous cardioverter-defibrillator and/or on the heart, a remaining battery capacity of a battery of the leadless pacemaker or the subcutaneous cardioverter-defibrillator.

Particularly, data may be transmitted from the leadless pacemaker to the ICD via said communication link. The ICD than communicates this data to the remote sever/computer.

Summarizing, the above-described aspect of the present invention allows to provide comprehensive therapy via the combination of a low voltage system, i.e. the leadless pacemaker, that is implanted in the heart of the patient (e.g. right or left ventricle) and a high voltage system, i.e. the subcutaneous ICD, that is implanted in a subcutaneous position, e.g. on the left side of the chest in the same patient.

Furthermore, the miniaturization of cardiac therapy devices and the desire to provide therapy without implanting transvenous leads generates new limits for functions of these miniaturized devices (e.g. leadless pacemakers). These limits are generated by significantly smaller batteries that can be used in such devices due to the volume requirements for the device itself. One example is the use of communication to body external devices via inductive or RF telemetry. Other examples are therapy options. For instance, it is currently technically not feasible to provide high voltage therapy to a heart using such a miniaturized device.

In this regard, the above-presented aspect of the present invention, particularly proposes to let the leadless pacemaker perform heart rate and rhythm analysis, and in case the therapy requires a high voltage solution, communicate unidirectionally with the subcutaneous ICD to request the delivery of a shock.

However, this technical solution always requires a communication link between the two devices, which adds additional current draw from the already size limited battery of the leadless pacemaker. Also, every communication link has the potential for electromagnetic or mechanical interference which might render the transferred information invalid. This can delay the delivery of therapy or in worst case even suppress therapy delivery. Therefore, according to a further aspect of the present invention, a cardiac pacemaking system is disclosed that comprises an implantable leadless pacemaker and an implantable subcutaneous cardioverter-defibrillator, wherein here both components, i.e. the leadless pacemaker and the subcutaneous ICD are configured to operate based on a predefined mode which generates a deterministic therapy approach, such that there is no need for a communication link between the two components. Preferably, such a communication link is completely absent.

According to this aspect of the present invention, after implantation of the entire system containing both devices (leadless pacemaker and subcutaneous ICD), or the implantation of the last component (leadless pacemaker or subcutaneous ICD) of the entire system (e.g. patient already had an leadless pacemaker implanted to treat a bradycardia and now requires a system upgrade to treat tachycardia, also) a follow-up is being performed. During this follow-up procedure both devices, i.e. the leadless pacemaker and the subcutaneous ICD, are programmed and adjusted to the specific needs of the patient. The deterministic approach presented in the application can be achieved by programming both devices with the comprehensive therapy set - independent of the ability to perform the specific therapy by one of the devices. In addition to the comprehensive therapy set, parameter of the comprehensive therapy set are labeled by an identifier which indicates which of the two devices is responsible for a specific therapy subset. An example of a therapy set is provided in the following table:

| Therapy | device leadless pacemaker - responsible | device ICD - responsible |
|---|---|---|
| Brady-pacing | 60bpm - leadless pacemaker | 60bpm - leadless pacemaker |
| ATP rate | 200 bpm | 200 bpm |
| ATP therapy | 3x Burst - leadless pacemaker | 3x Burst - leadless pacemaker |
| Shock | After 3 ATP - ICD | After # ATP - ICD |
| Post shock pacing | 20 cycles after shock - leadless pacemaker | 20 cycles after shock - leadless pacemaker |

In the provided example both devices, the leadless pacemaker and the subcutaneous ICD, "know" what the therapy options are and which device is providing which part of the therapy. So if the intrinsic heart rate of the patient would exceed 200bpm, the leadless pacemaker would deliver 3 series of ATP burst therapy. If this therapy would not stop the tachyarrythmia the leadless pacemaker would stop and "wait" for the ICD to deliver a shock. Immediately after the shock the leadless pacemaker would start providing 20 cycles post shock pacing. As expected, it is not sufficient for both devices to "know" what the own task as well as the task of the other device is, but both devices need to be able to work from the same time basis.

Therefore, according to an embodiment a time synchronization is implemented. For this, different methods may be used:
According to a first embodiment, a common "clock" is used which is the patient's intrinsic heart rate. Here, in order to provide adequate therapy, both devices are configured to sense the intrinsic rhythm of the patient's heart.

According to a further embodiment both devices are configured to detect a specific therapy pattern of the other device, e.g. the subcutaneous ICD may be configured to detect the pacing therapy of the leadless pacemaker and count the number of therapies (e.g. delivered pacing pulses). On the other side, the leadless pacemaker may be configured to detect the delivery of the high voltage shock by the ICD and begin post shock pacing.

According to a further embodiment, fixed time intervals may be used after a criterion for therapy delivery is initiated (e.g. the ICD can just wait for a specific amount of time, e.g. "X" seconds, before charging the output capacitors after the tachycardia is detected). In case the same tachycardia is still ongoing after said specific amount of time, it knows that the leadless pacemaker was not successful terminating the tachycardia and therefore delivers the shock therapy.

This aspect of the present invention has the advantage that both therapy devices do not need to communicate with each other via a body area network. Also, it reduces the delay or even loss of therapy due to a disturbed communication link or failure of the communication link between the two devices.

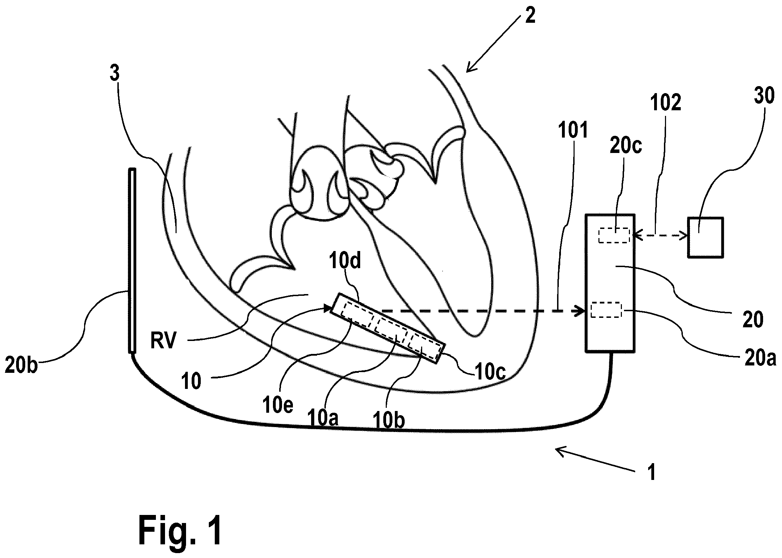

- Fig. 1: shows a schematical representation of a cardiac pacemaking device according to the invention.

Figure 1 shows a cardiac pacemaking system 1 comprising an implantable leadless pacemaker (leadless pacemaker) 10 and a subcutaneous implantable cardioverter-defibrillator 20 as well as an unidirectional communication link 101 between the leadless pacemaker 10 and the subcutaneous ICD 20. The leadless pacemaker 10 is configured to establish this communication link with help of a suitable transmitter means 10e, while the ICD 20 is configured to receive output signals of the leadless pacemaker 10 by means of a suitable receiver means 20a.

Particularly, the leadless pacemaker 10 is configured to apply low voltage electrical pacing pulses to the heart 2, particularly in order to provide anti-bradycardia pacing, anti-tachycardia pacing (ATP), and post shock pacing, i.e. pacing after an electrical high voltage stimulation by the ICD 20. In contrast thereto, the subcutaneous ICD 20 is configured to apply high voltage electrical stimulation to the heart 2 for delivering a shock therapy to the heart, e.g. for providing defibrillation of the heart 2.

Further, the leadless pacemaker 10 is configured to sense the patient's heart rhythm, and to send an output signal to the subcutaneous ICD 20 via said communication link 101 when the leadless pacemaker 10 detects a heart rhythm that requires said electrical stimulation of the subcutaneous cardioverter-defibrillator 20. The subcutaneous ICD 20 in turn is configured to apply said electrical stimulation to the heart 2 when it receives said output signal of the leadless pacemaker 10.

Particularly, the leadless pacemaker 10 may comprise a hermetically sealed housing 10d enclosing a pulse generator 10b for generating said pacing pulses and a battery 10a for supplying energy to the pulse generator 10b. The leadless pacemaker 10 may further comprise fastening means provided on the housing 10d for fastening the leadless pacemaker 10 to the chamber/ventricle (here the right ventricle RV), and a pacing electrode 10c provided e.g. on an end of the housing 10d of the leadless pacemaker 10 for applying the electrical stimulation to the heart 2. Regarding the pacemaker 10 the notion leadless means that the electrode 10c of the leadless pacemaker 10 is not connected via an external lead to the housing 10d of the pacemaker 10 but is provided on the housing 10d.

Further, particularly, the ICD 20 does not comprise a direct electrode connection to the myocardium 3, but an electrode 20b that may be positioned adjacent to the sternum of the patient, wherein the ICD 20 may be implanted subcutaneously on the left side of the chest of the patient. Advantageously, according to the invention, the ICD 20 does not need/comprise a sensing means for sensing the heart rhythm of the heart 2 of the patient, but receives triggering output signals via the communication link 101 when high voltage therapy is necessary as described herein.

Further, in order to monitor the system 1, the ICD 20 is configured to establish a further communication link 102, particularly an RF link, to a server/computer 30 for uploading data about therapy conducted by the leadless pacemaker and/or ICD as well as status data, for instance a remaining capacity of the battery 10a of the leadless pacemaker. For establishing this link, the ICD 20 may comprise a suitable transmitter means 20c.

## Claims

**1.** A cardiac pacemaking system (1), comprising:
an implantable leadless pacemaker (10) that is configured to be implanted into a chamber of a patient's heart (2), and
an implantable subcutaneous cardioverter-defibrillator (20), wherein
the leadless pacemaker (10) is configured to apply electrical pacing pulses to the heart (2), particularly in order to provide antibradycardia pacing, and wherein
the subcutaneous cardioverter-defibrillator (20) is configured to apply electrical stimulation to the heart (2) for providing defibrillation of the heart (2), and wherein the leadless pacemaker (10) is configured to sense the patient's heart rhythm, and wherein
the cardiac pacemaking system (1) comprises a communication link (101) between the leadless pacemaker (10) and the subcutaneous cardioverter-defibrillator (20), wherein
the leadless pacemaker (10) is configured to send an output signal to the subcutaneous cardioverter-defibrillator (20) via said communication link (101) in an unidirectional fashion when the leadless pacemaker (10) detects a heart rhythm that requires said electrical stimulation of the subcutaneous cardioverter-defibrillator (20), and wherein
the subcutaneous cardioverter-defibrillator (20) is configured to apply said electrical stimulation to the heart (2) when the subcutaneous cardioverter-defibrillator (20) receives said output signal.

**2.** The pacemaking system according to claim 1, wherein the subcutaneous cardioverter-defibrillator (20) is configured to merely receive information about the patient's heart (2) via said unidirectional communication link (101).

**3.** The cardiac pacemaking system according to claims 1 or 2, wherein the leadless pacemaker (10) is further configured to provide heart rhythm classification of a sensed heart rhythm, particularly whether the sensed heart rhythm comprises a rate that is too low, normal, or too high; and/or whether said sensed heart rhythm requires electrical therapy by the subcutaneous cardioverter-defibrillator (20); and/or whether said sensed heart rhythm requires electrical therapy from the leadless pacemaker (10).

**3.** The cardiac pacemaking system according to one of the preceding claims, wherein the leadless pacemaker (10) is further configured to identify a source of a heart rhythm disturbance, particularly whether said heart rhythm disturbance originates from atrial or ventricular arrhythmia.

**4.** The cardiac pacemaking system according to one of the preceding claims, wherein the leadless pacemaker (10) is configured to apply electrical pacing pulses to the heart for providing at least one of:
- antibradycardia pacing,
- anti-tachycardia pacing (ATP),
- post shock pacing.

**5.** The cardiac pacemaking system according to one of the preceding claims, wherein the leadless pacemaker (10) is configured to measure cardiac output.

**6.** The cardiac pacemaking system according to one of the preceding claims, wherein the subcutaneous cardioverter-defibrillator (20) is configured to transmit data to a remote server (30) via a further communication link (102).

**7.** The cardiac pacemaking system according to claim 6, wherein the subcutaneous cardioverter-defibrillator (20) is configured to transmit said data to said remote server (30) upon request of the leadless pacemaker (10), which is configured to transmit said request to the subcutaneous cardioverter-defibrillator (20) via said communication link (101).

**8.** The cardiac pacemaking system according to claim 6 or 7, wherein said data is at least one of: data about electrical stimulation applied to the heart by the leadless pacemaker (10) and/or by the subcutaneous cardioverter-defibrillator (20), status information on the leadless pacemaker (10) and/or on the subcutaneous cardioverter-defibrillator (20) and/or on the heart (2), the remaining battery capacity of a battery (10a) of the leadless pacemaker (10), and/or by the subcutaneous cardioverter-defibrillator (20).
